# EUROPEAN PATENT SPECIFICATION

(11) **EP 2 254 532 B1**
(45) Date of publication and mention of the grant of the patent: **15.08.2012**
(21) Application number: 09710172.9
(22) Date of filing: 04.02.2009
(51) Int. Cl.: A61F 5/01

(54) **DEVICE FOR THE POST-OPERATIVE OR CONSERVATIVE TREATMENT OF THE HIP**
VORRICHTUNG ZUR POSTOPERATIVEN ODER KONSERVATIVEN BEHANDLUNG DER HÜFTE
DISPOSITIF POUR LE TRAITEMENT POSTOPÉRATOIRE OU CONSERVATEUR DE LA HANCHE

(30) Priority: 12.02.2008 IT VR20080017
(43) Date of publication of application: 01.12.2010
(73) Proprietor: F.G.P. Srl, 37062 Dossobuono (VR) (IT)
(72) Inventor: FERRIGOLO, Moreno, I-37062 Dossobuono (IT); TURRINI, Alberto, I-37062 Dossobuono (vr) (IT)
(74) Representative: Sandri, Sandro
(86) International application number: PCT/IB2009/050452
(87) International publication number: WO 2009/101547

(56) References cited:
- US-A- 4 881 532
- US-A- 6 039 707
- US-A1- 2003 100 854
- US-A1- 2004 024 340
- US-A1- 2006 015 049

## Description

### TECHNICAL FIELD

This invention concerns a hip device or brace for the postoperative or conservative treatment of diseases that involve the risk of hip dislocation.

More specifically, this invention refers to a hip brace for the postoperative or conservative treatment of hip diseases which has the substantial advantage of the greater anatomical adaptability that the elements of the brace present with respect to known solutions. In fact, the gripping pats of the braces currently available on the market are made from plastic materials which, once formed, are difficult to "finely" adapt to the patient's anatomy.

The hip brace according to the invention can on the other hand be adapted to various situations and conditions, as well as being very simple in design and thus very inexpensive to produce.

This invention can be applied in the production sector of accessories for orthopaedic products and braces mainly used in conservative, post-traumatic, rehabilitation and post-operative treatment.

### BACKGROUND ART

It is known that for diseases involving dislocation of the hip it is sometimes necessary to limit or to partially or totally block the joint. The need to block the joint may be necessary following surgery or trauma or also due to congenital defects.

For the total or partial blocking of the joint, necessary in the cases described above, braces are used which generally consist of an upper part, shaped to wrap around the patient's waist or pelvis, and a femoral part, shaped to wrap around the upper part of the thigh. The two pelvis-gripping and thigh-gripping parts are generally connected to each other by means of a mechanical joint with angular movement, which allows total or partial and controlled blocking of the hip joint.

Known hip braces present so-called grippers which ensure firm gripping of the upper part of the brace around the pelvis and of the lower part around the thigh.

Constructed in this way, traditional braces allow control of the flexion-extension and abduction-adduction movements, as well as limitation of the intra-extra rotation and stabilization of the hip joint.

However, known hip braces present gripper parts made from plastic materials which, once formed, are difficult to "finely" adapt to the patient's anatomy, causing, especially in the long-term, considerable discomfort for the patient.

Another problem encountered with known braces is caused by the influence that the use of this material has on the weight of the structure. In fact, in order to have the necessary mechanical resistance to the strain involved, the solutions made using this material must be fairly thick and heavy with an obvious negative effect on the comfort of the person wearing the brace, especially if one considers the combination of this factor with the previous drawback relative to the difficult adaptability of plastic to the patient's anatomy.

In addition, traditional braces are equipped with a joint with limited functional features, since only with difficulty and impracticality can they perform the dual task of ensuring freedom of flexion and extension movements of the joint within the established range of motion and of inducing the consequent abduction movement. Document US 2003/100854 A1 discloses a joint rod for a hip support comprising a first element suitable for being associated with a pelvis grip, and a second element suitable for being associated with a thigh grip, reciprocally joined by a hinge for a rotating movement in a first flexure-extension plane, and by an articulated joint for movement in a second abduction-adduction plane, wherein the articulated joint is connected to an activating device which causes a movement of the second element of the jointed rod in the abduction-adduction plane with the rotation of said second element with respect to the first element. Document US-A-6039707 discloses a pelvic support and walking assistance device including a trunk support member and a thigh lifting member coupled to each other at a pivot point positioned opposite a hip joint of a user. The device functions to prevent pelvic sway. With a spring coupled with said pivot joint, the device provides a thigh lifting torque to partially compensate for proximal leg weakness. The spring assistance pivot device includes a winding device that can be adjusted in use for varying forward torque for varying situations. Document US 2004/024340 A1 discloses a hip orthosis including a hip engaging unit that can be secured to the contours of a hip. An appendant orthotic member is formed to extend diagonally about and to be fixed to a leg. A connector assembly, with a support plate with a curved configuration at an anchor location to permit adjustment, interconnects the hip engaging member and the appendant member and includes an articulated joint member to control flexion, extension, abduction and adduction.

### DESCRIPTION OF THE INVENTION

This invention proposes to provide a hip device or brace, for the postoperative or conservative treatment of diseases that involve the risk of dislocation of the hip, able to eliminate or at least reduce the above-mentioned drawbacks through means aimed at greater adaptability of the brace to the patient's anatomy and reduction of the weight of the brace, in order to make the device more ergonomic in general.

The invention also proposes to provide the possibility of excluding the abduction mechanism, thus giving the brace the simple task of supporting and guiding, or of blocking this mechanism at a certain limit.

This is achieved by means of a hip brace presenting the features described in the main claim.

The dependent claims described advantageous embodiments of the invention.

The proposed aims are achieved, according to the invention, by a hip brace for the postoperative or conservative treatment of diseases that involve the risk of dislocation of the hip, one of the features being the possibility of selecting the extent of the required abduction of the joint very easily and with precision.

In addition, the brace also foresees the possibility of blocking the abduction at 35°, which is a further safety means to prevent dislocation.

The brace according to the invention also foresees the presence of a mechanism which makes it possible to advance or delay the intervention of the pin that induces abduction, providing an additional adjustment to favour adaptation of the brace functioning to individual biomechanics.

It is also possible to exclude the abduction mechanism, thus giving the brace the simple task of supporting and guiding, or to block this mechanism at a certain limit.

### DESCRIPTION OF THE DRAWINGS

Other features and advantages of the invention will become evident on reading the description given below of one embodiment of the invention, provided as a non-binding example, with the help of the accompanying drawings in which:

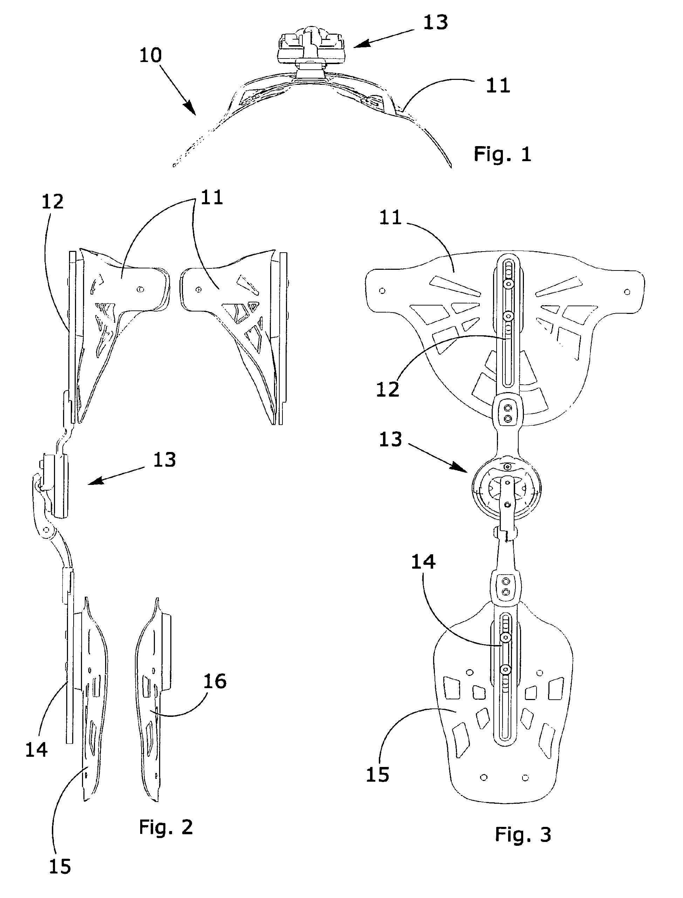

- figures 1, 2, 3 represent schematic views of, respectively, the front profile, the side profile, the front view and the side view of a part of the femoral support and the respective pelvic side part of the brace according to the invention;

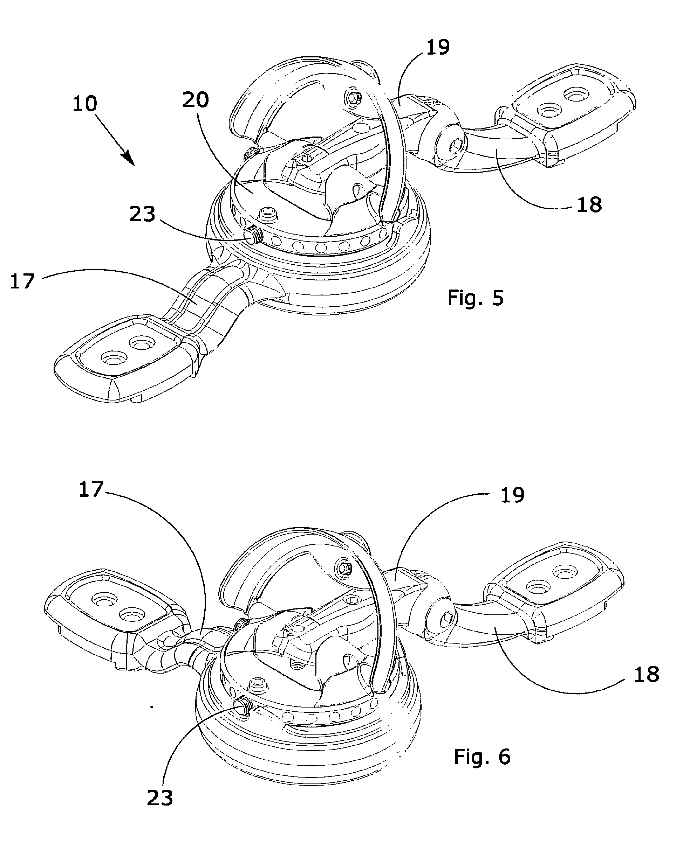

- figures 5 and 6 show the schematic view of the joint and the relative cover of the brace according to the invention in two angular positions at 0° and at 90°;
- figures 7 to 10 show schematic views of the joint of the brace according to the invention in the maximum extension position, rotated at 90° and in cross-section rotated at 90°;

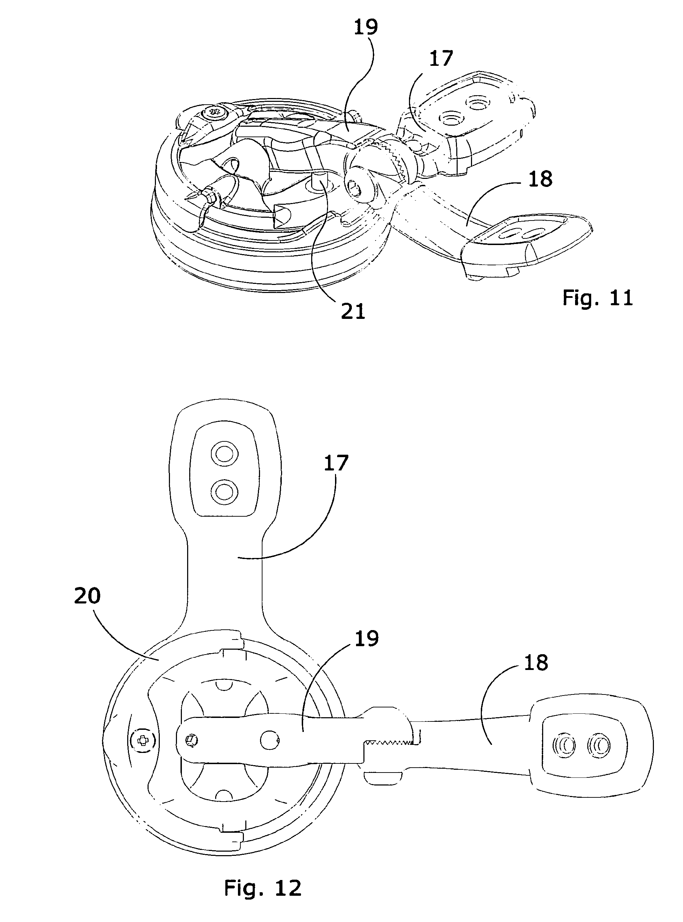

- figures 11 and 12 show an axonometric and frontal view of the joint, and bent at 90°;
- figures 13, 14 and 15 represent views of the brace with the joint at 45°;
- figure 16 is an exploded schematic view of the brace;

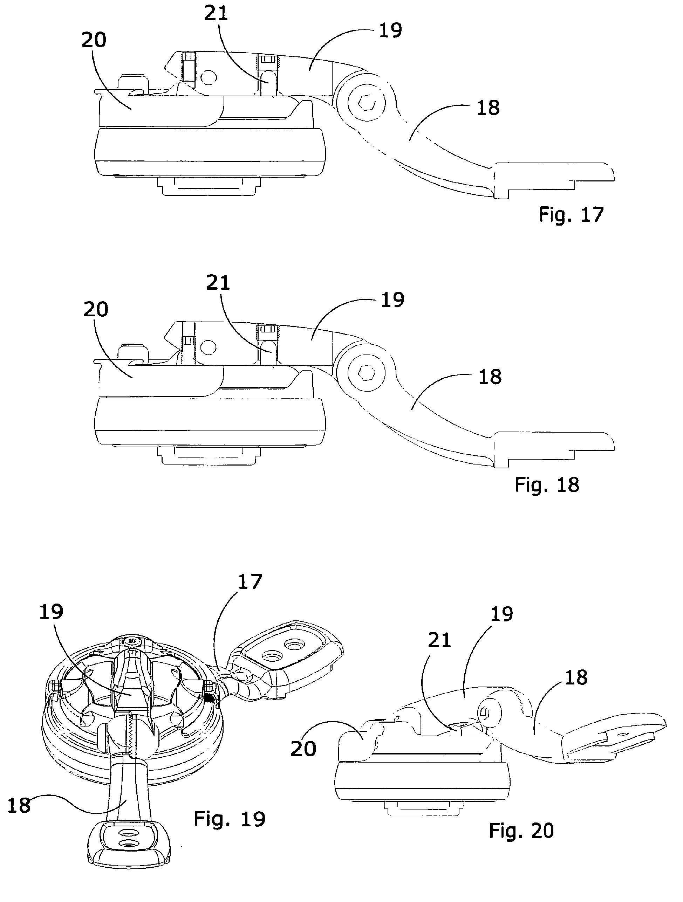

- figures 17 and 18 are schematic views of the joint with the rod disabled and the pin-cam unit and with the rod blocked;
- figures 19 and 20 are views of the joint rotated at 45°;

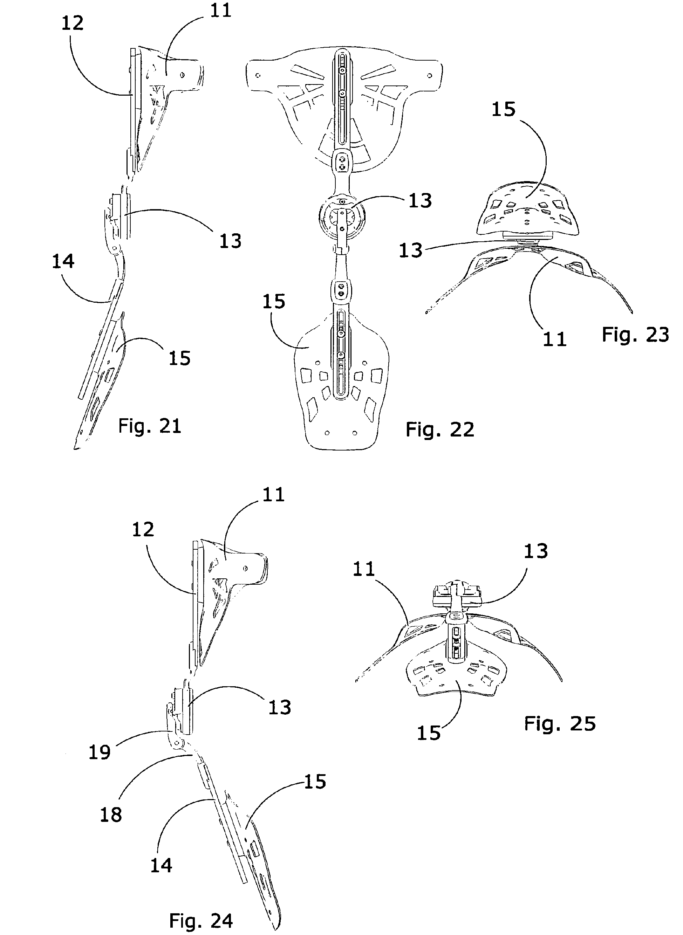

- figures 21, 22 and 23 are overall views of the brace joint rotated at +20°;
- figures 24 and 25 are overall views of the brace joint rotated at -20°.

### DESCRIPTION OF ONE EMBODIMENT OF THE INVENTION

The brace according to the invention, mainly if not exclusively used in the orthopaedic sector and indicated overall with the reference number 10, substantially consists of a double iliac support 11, that is to say one for the right part and the other for the left part of the pelvis, an upper joint position adjustment rod 12, an articulated joint 13, a lower joint position adjustment rod 14 and an inner 15 and outer femoral support 16.

The brace therefore consists of two sectors, connected by the articulated joint 13 to be positioned at the level of the affected hip joint, and of a system of supports and straps (not shown in the figures) connected to the articulated joint and designed to make the brace fit firmly to the patient's body.

The supports 11, 15 and 16 are made from metal, and specifically from aluminium alloy (e.g. peralluman H111) which has the dual purpose of guaranteeing a firm grip to ensure the correct position of the joint is maintained (and thus continuous efficacy of the brace) and of favouring the adaptability of the structure to the patient's anatomy.

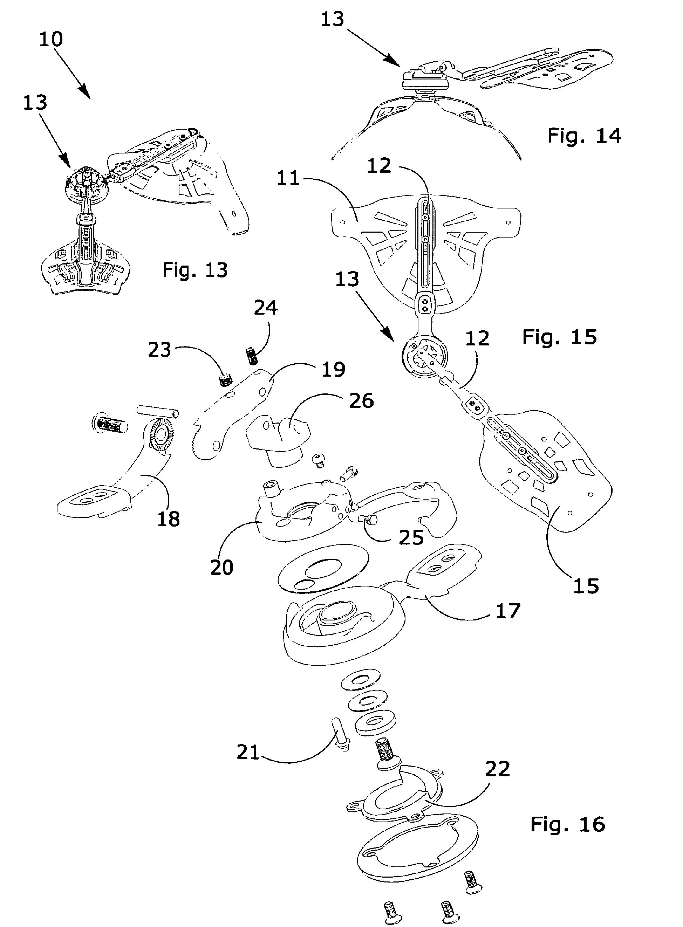

The main components of the joint (those that are designed to carry out the functions of the brace) are completed by the components in the exploded view shown in figure 16, that is to say a connecting element 17 of the upper rod, two fixing elements 18 and 19 of the lower rod, a ring nut 20 for adjustment of the range of motion, a ball pin 21 designed to induce the abduction movement of the hip during the flexion phase, an abduction adjustment cam 22, an abduction advance/delay adjustment grub screw 23, an abduction blocking grub screw 24, a blocking pin 25 and a hinge bracket 26.

In addition to fixing, the connecting element 17 of the upper rod 12 also allows adjustment of the R.O.M. (Range Of Motion) thanks to its geometrical shape, and in fact the pins positioned in the ring nut 20 are blocked by its projections.

The fixing elements 18 and 19 of the lower rod 14 are separate and can be fixed to each other by a locking screw. The particular shape of the surfaces shows the possibility of varying the relative inclination of the two elements in order to ensure correct alignment of the femoral supports with the patient's joint.

The fixing element 19 is also involved in inducing the abduction movement of the hip during flexion, as will be better explained below in the description of how the brace functions.

It should be stressed that this fixing element 19 is designed and constructed with a surface shape that ensures a safety stop in abduction at around 35°.

The circumference of the ring nut 20 for adjustment of the R.O.M. presents a series of holes in which it is possible to house the pins 21, which, depending on the hole in which they are positioned, ensure the mechanical stop of the flexion or extension of the hip joint.

The function of the ball pin 21 is to induce the abduction movement of the hip during the flexion phase.

The abduction adjustment cam 22 is shaped so as to guarantee the possibility of inducing the abduction movement of the coxo-femoral joint, and also to offer the possibility of choosing three different types of intervention (respectively, neutral, abduction at 15° and abduction at 25°).

From a functional point of view, the articulated joint 13 therefore has the dual purpose of ensuring freedom of movement in flexion and extension of the hip joint within the established R.O.M. limits and of inducing the abduction movement connected with the articulated joint.

More specifically, the "flexion" of the brace is guaranteed by hinged kinematic torque provided by the connecting element 17 and by the bracket 26 which is in turn hinged to the fixing element 18.

The movement of the bracket 26 with respect to the connecting element 17 drags the pin 21 which will move relative to the cam 22 which is integral with the connecting element 17.

The movement of the pin 21 is thus conditioned by the surface which the ball encounters, which can be a constant-slope cam with maximum elevation corresponding to 25° abduction, or 15° or, finally, a neutral 0° constant-slope cam.

The pin 21 will move along the neck of the ring nut 20 on which the fixing element 18 is hinged, creating a thrust by the pin 21 on the fixing elements 18 and 19, thus inducing abduction.

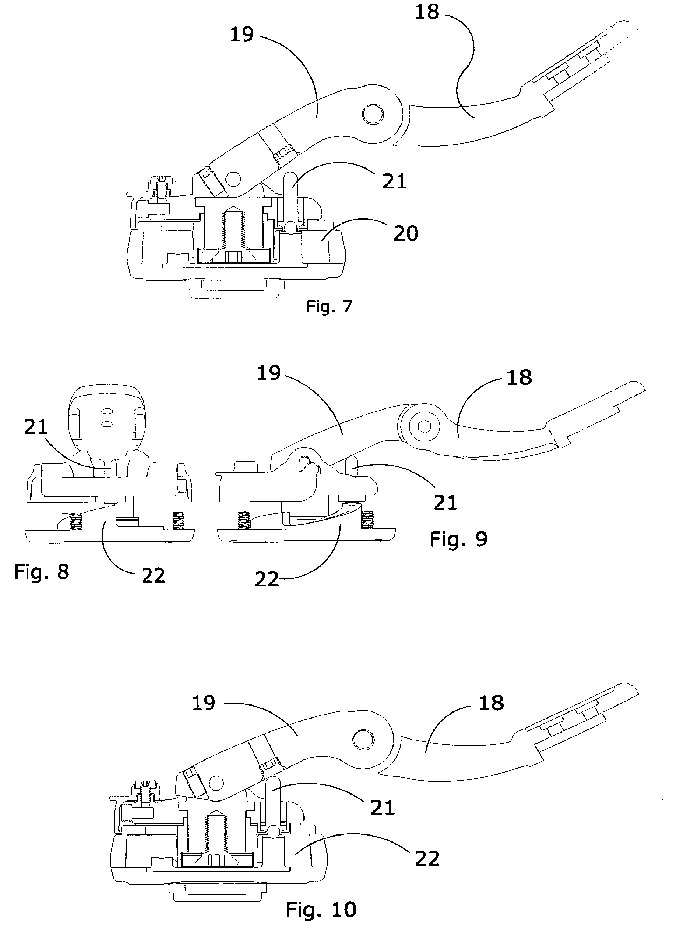

Figures 9 and 10 show the maximum elevation position of the pin 21.

The articulated joint is thus designed to provide a certain range of abduction, which can be determined by the choice of the cam 22 on which the pin 21 slides, in relation to the flexion/extension movement.

In addition to this direct relationship, the articulated joint 13 is designed to allow adjustment of this abduction: in fact by turning the grub screw 23, it is possible to delay or advance the abduction.

The grub screw 23 is in fact positioned in correspondence with the ideal sliding axis of the pin 21 and its end surface is in direct contact with that of the pin 21.

By screwing the grub screw 23 into its housing beyond the neutral position, it is thus already possible to determine a certain degree of abduction.

By unscrewing the grub screw 23 with respect to the neutral position, it is instead possible to delay induction of the abduction movement which will be proportional to the distance between the end surface of the grub screw 23 and the head of the pin 21 and the inclination of the cam 22 being used.

Designed in this way, the structure of the mechanism has built-in safety during abduction: the end surface protruding beyond the hinge of the fixing element 19 is inclined in such a way that it does not allow abduction greater than 45°.

The invention is described above with reference to a preferred embodiment. It is nevertheless clear that the invention is susceptible to numerous variations which lie within the scope of its disclosure, in the framework of technical equivalents.

## Claims

1. An orthopaedic brace (10) consisting of two iliac supports (11), that is to say one for the right part and the other for the left part of the pelvis, and inner and outer femoral supports (15, 16), these iliac (11) and femoral supports (15, 16) being connected by an articulated joint (13), whereby the articulated joint (13) comprises at least one pin or grub screw (21) engaging with an abduction adjustment cam (22), and means of abduction adjustment, advance, delay and blocking (23, 25, 27), and whereby the iliac supports (11) are fixed to the articulated joint (13) by means of a connecting element (17) with an upper rod (12), while the lower femoral supports (15, 16) are connected to the articulated joint by means of fixing elements (18, 19) with a lower rod (14), **characterised in that** the articulated joint (13) comprises a ring nut (20) for adjustment of the Range of Motion, a ball pin (21) designed to induce the abduction movement of the hip during the flexion phase, an abduction adjustment cam (22), an abduction advance/delay adjustment grub screw (23) an abduction blocking grub screw (24), a blocking pin (25) and a hinge bracket(26).

2. An orthopaedic brace (10) according to claim 1, **characterised in that** the supports (11, 15, 16) are made from metal.

3. An orthopaedic brace (10) according to any of the foregoing claims, **characterised in that** it consists of two sectors connected together by the articulated joint (13) to be positioned at the level of the affected hip joint, and of a system of supports and straps connected to the articulated joint and designed to make the brace fit firmly to the patient's body.

4. An orthopaedic brace (10) according to any of the foregoing claims, **characterised in that** the supports (11, 15 and 16) are made from metal, and specifically from aluminium alloy (e.g. peralluman H111) which has the dual purpose of guaranteeing a firm grip to ensure the correct position of the joint is maintained (and thus continuous efficacy of the brace) and of favouring the adaptability of the structure to the patient's anatomy.

5. An orthopaedic brace (10) according to any of the foregoing claims, **characterised in that**, in addition to fixing, the connecting element (17) of the upper rod (12) also allows adjustment of the R.O.M. (Range Of Motion) thanks to its geometrical shape, and in fact the pins positioned in the ring nut (20) are blocked by its projections.

6. An orthopaedic brace (10) according to any of the foregoing claims, **characterised in that** the fixing elements (18 and 19) of the lower rod (14) are separate and can be fixed to each other by a locking screw.

7. An orthopaedic brace (10) according to any of the foregoing claims, **characterised in that** the particular shape of the surfaces of the fixing elements (18, 19) shows the possibility of varying the relative inclination of the two elements in order to ensure correct alignment of the femoral supports with the patient's joint.

8. An orthopaedic brace (10) according to any of the foregoing claims, **characterised in that** the fixing element (19) is also involved in inducing the abduction movement of the hip during flexion.

9. An orthopaedic brace (10) according to any of the foregoing claims, **characterised in that** the fixing element (19) is designed and constructed with a surface shape that ensures a safety stop in abduction at around 35°.

10. An orthopaedic brace (10) according to any of the foregoing claims, **characterised in that** the circumference of the ring nut (20) for adjustment of the R.O.M. presents a series of holes in which it is possible to house the pins (21), which, depending on the hole in which they are positioned, ensure the mechanical stop of the flexion or extension of the hip joint.

11. An orthopaedic brace (10) according to any of the foregoing claims, **characterised in that** the ball pin (21) makes it possible to induce the abduction movement of the hip during the flexion phase.

12. An orthopaedic brace (10) according to any of the foregoing claims, **characterised in that** the abduction adjustment cam (22) is shaped so as to guarantee the possibility of inducing the abduction movement of the coxo-femoral joint, and also to offer the possibility of choosing three different types of intervention (respectively, neutral, abduction at 15° and abduction at 25°).

## Patentansprüche

1. Orthopädische Klammer (10) bestehend aus zwei Hüftstützen (11), das heißt eine für den rechten Teil und die andere für den linken Teil des Beckens, sowie innere und äußere Oberschenkelstützen (15, 16), wobei diese Hüft- (11) und Oberschenkelstützen (15, 16) durch ein artikuliertes Gelenk (13) verbunden sind, wobei das artikulierte Gelenk (13) mindestens einen Stift oder Gewindestift (21) aufweist, der in einen Abduktion-Einstellnocken (22) eingreift, sowie Vorrichtungen für die Abduktionseinstellung (vorwärts, Verzögerung und Blockierung), und wobei die Hüftstützen (11) am artikulierten Gelenk (13) mit einem Verbindungselement (17) an der oberen Stange (12) fixiert sind, während die unteren Oberschenkelstützen (15, 16) am artikulierten Gelenk mit Fixierelementen (18, 19) an der unteren Stange verbunden sind,
**dadurch gekennzeichnet, dass**
das artikulierte Gelenk (13) eine Ringmutter (20) zur Einstellung des Bewegungsbereichs (R.O.M.) aufweist, einen Kugelbolzen (21), der dazu dient, die Abduktionsbewegung der Hüfte in der Flexionsphase zu veranlassen, mit einem Abduktions-Einstellnocken (22), einem Gewindestift (23) zum Einstellen der Vorwärts- oder Verzögerungs-Abduktion, einem Gewindestift (24) zum Blockieren der Abduktion, einem Blockierstift )25) und einem Scharnierbügel (26).

2. Orthopädische Klammer (10) gemäß Anspruch 1, **dadurch gekennzeichnet, dass** die Stützen (11, 15, 16) aus Metall gefertigt sind.

3. Orthopädische Klammer (10) gemäß einem der vorhergehenden Ansprüche, **dadurch gekennzeichnet, dass** sie aus zwei durch das artikulierte Gelenk (13) verbundenen Abschnitten besteht, die an das betroffene Hüftgelenk angepasst werden, sowie aus einem System von Stützen und Gurten, die am artikulierten Gelenk befestigt sind und dazu dienen, die Klammer fest am Körper des Patienten anzubringen.

4. Orthopädische Klammer (10) gemäß einem der vorhergehenden Ansprüche, **dadurch gekennzeichnet, dass** die Stützen (11, 15 und 16) aus Metall und zwar spezifisch aus Aluminiumlegierung (z.B. Peraluman H111) gefertigt sind, wodurch der doppelte Zeck erfüllt wird, einen festen Halt zu gewähren, um die richtige Position des Gelenks (und damit die Wirksamkeit der Klammer) aufrecht zu erhalten, und die Anpassungsfähigkeit der Struktur an die Anatomie des Patienten zu begünstigen.

5. Orthopädische Klammer (10) gemäß einem der vorhergehenden Ansprüche, **dadurch gekennzeichnet, dass** das Verbindungselement (17) der oberen Stange (12) nicht nur die Fixierung, sondern aufgrund seiner geometrischen Form auch die Einstellung des Bewegungsbereichs ermöglicht, und dass seine Vorsprünge auch die in der Ringmutter (20) steckenden Stifte blockieren.

6. Orthopädische Klammer (10) gemäß einem der vorhergehenden Ansprüche, **dadurch gekennzeichnet, dass** die Fixierelemente (18 und 19) der unteren Stange (14) getrennt sind und mit einer Feststellschraube aneinander befestigt werden können.

7. Orthopädische Klammer (10) gemäß einem der vorhergehenden Ansprüche, **dadurch gekennzeichnet, dass** die spezifische Oberflächengestalt der Fixierelemente (18, 19) ermöglicht, die relative Neigung der bedien Elemente zueinander zu variieren, um die richtige Ausrichtung der Oberschenkelstützen mit dem Gelenk des Patienten zu gewährleisten.

8. Orthopädische Klammer (10) gemäß einem der vorhergehenden Ansprüche, **dadurch gekennzeichnet, dass** das Fixierelement (19) auch daran beteiligt ist, die Abduktionsbewegung der Hüfte im Flexionsstadium zu veranlassen.

9. Orthopädische Klammer (10) gemäß einem der vorhergehenden Ansprüche, **dadurch gekennzeichnet, dass** das Fixierelement (19) mit einer Oberfläche konfiguriert und gebaut ist, die bei Abduktion um etwa 35 Grad einen Sicherheits-Stopp gewährleistet.

10. Orthopädische Klammer (10) gemäß einem der vorhergehenden Ansprüche, **dadurch gekennzeichnet, dass** der Umfang der Ringmutter (20) für die Einstellung des Bewegungsbereichs eine Reihe von Löchern aufweist, in denen Stifte (21) aufgenommen werden können, die je nach dem Loch, in das sie gesteckt werden, den mechanischen Stopp der Flexion oder Dehnung des Hüftgelenks gewährleisten.

11. Orthopädische Klammer (10) gemäß einem der vorhergehenden Ansprüche, **dadurch gekennzeichnet, dass** der Kugelbolzen (21) ermöglicht, die Abduktionsbewegung der Hüfte in der Flexionsphase veranlasst.

12. Orthopädische Klammer (10) gemäß einem der vorhergehenden Ansprüche, **dadurch gekennzeichnet, dass** der Abduktions-Einstellnocken (22) so gestaltet ist, dass er die Möglichkeit gewährt, die Abduktionsbewegung des Hüftgelenks zu veranlassen und die Möglichkeit gewährt, drei verschiedene Arten von Intervention zu wählen: neutral, Abduktion um 15 Grad und Abduktion um 25 Grad.

## Revendications

1. Appareil orthopédique (10) constitué de deux supports iliaques (11), c'est-à-dire un pour la partie droite et l'autre pour la partie gauche du bassin, et des supports fémoraux intérieur et extérieur (15, 16), ces supports iliaques (11) et fémoraux (15, 16) étant raccordés par une articulation (13), l'articulation (13) comprenant au moins une cheville ou vis sans tête (21) s'engageant avec une came de réglage d'abduction (22), et des moyens de réglage, d'avancement, de retardement et de blocage d'abduction (23, 25, 27), et les supports iliaques (11) étant fixés à l'articulation (13) au moyen d'un élément (17) de raccordement à une tige supérieure (12), tandis que les supports fémoraux inférieurs (15, 16) sont raccordés à l'articulation au moyen d'éléments (18, 19) de fixation à une tige inférieure (14), **caractérisé en ce que** l'articulation (13) comprend un écrou en anneau (20) pour le réglage de la plage de mouvement, un boulon à rotule (21) conçu pour induire le mouvement d'abduction de la hanche pendant la phase de flexion, une came de réglage d'abduction (22), et une vis sans tête (23)de réglage d'avancement/de retardement d'abduction, une vis sans tête (24) de blocage d'abduction, une cheville de blocage (25) et une attache d'articulation (26).

2. Appareil orthopédique (10) selon la revendication 1, **caractérisé en ce que** les supports (11, 15, 16) sont fabriqués à partir d'un métal.

3. Appareil orthopédique (10) selon l'une quelconque des revendications précédentes, **caractérisé en ce qu'**il est constitué de deux secteurs raccordés l'un à l'autre par l'articulation (13) à positionner au niveau de l'articulation de hanche affectée, et d'un système de supports et de bandes raccordé à l'articulation et conçue pour que l'articulation soit solidement maintenue sur le corps du patient.

4. Appareil orthopédique (10) selon l'une quelconque des revendications précédentes, **caractérisé en ce que** les supports (11, 15 et 16) sont fabriqués à partir d'un métal, et spécifiquement à partir d'un alliage d'aluminium (par exemple du peralluman H111) qui a le double but de garantir une préhension ferme pour assurer le positionnement correct de l'articulation (et donc une efficacité permanente de l'appareil orthopédique) et de favoriser la capacité d'adaptation de la structure à l'anatomie du patient.

5. Appareil orthopédique (10) selon l'une quelconque des revendications précédentes, **caractérisé en ce que**, en plus de la fixation, l'élément de raccordement (17) de la tige supérieure (12) permet également le réglage de la plage de mouvement grâce à sa forme géométrique, et en fait les chevilles positionnées dans l'écrou en anneau (20) sont bloquées par ses saillies.

6. Appareil orthopédique (10) selon l'une quelconque des revendications précédentes, **caractérisé en ce que** les éléments de fixation (18 et 19) de la tige inférieure (14) sont séparés et peuvent être fixés l'un à l'autre par une vis de blocage.

7. Appareil orthopédique (10) selon l'une quelconque des revendications précédentes, **caractérisé en ce que** la forme particulière des surfaces des éléments de fixation (18, 19) représente la possibilité de faire varier l'inclinaison relative des deux éléments afin d'assurer un alignement correct des supports fémoraux avec l'articulation du patient.

8. Appareil orthopédique (10) selon l'une quelconque des revendications précédentes, **caractérisé en ce que** l'élément de fixation (19) intervient également dans le mouvement d'abduction de la hanche pendant la flexion.

9. Appareil orthopédique (10) selon l'une quelconque des revendications précédentes, **caractérisé en ce que** l'élément de fixation (19) est conçu et construit avec une forme de surface qui assure une butée de sécurité pour l'abduction à environ 35°.

10. Appareil orthopédique (10) selon l'une quelconque des revendications précédentes, **caractérisé en ce que** la circonférence de l'écrou en anneau (20) pour le réglage de la plage de mouvement présente une série de trous dans lesquels il est possible de loger des chevilles (21) qui assure, en fonction du trou dans lequel elles sont positionnées, la butée mécanique de la flexion ou de l'extension de l'articulation de la hanche.

11. Appareil orthopédique (10) selon l'une quelconque des revendications précédentes, **caractérisé en ce que** le boulon à rotule rend possible de provoquer un mouvement d'abduction de la hanche pendant la phase de flexion.

12. Appareil orthopédique (10) selon l'une quelconque des revendications précédentes, **caractérisé en ce que** la came de réglage d'abduction (22) est doté d'une forme permettant de garantir la possibilité d'induire le mouvement d'abduction de l'articulation coxo-fémorale, et donc d'offrir la possibilité de choisir trois types différents d'intervention (respectivement, neutre, abduction à 15° et abductions à 25°).
